# DEMANDE DE BREVET EUROPEEN

(11) **EP 4 144 341 A1**
(43) Date de publication de la demande: **08.03.2023**
(21) Numéro de dépôt: 22193515.8
(22) Date de dépôt: 01.09.2022
(51) Int. Cl.: A61K 8/44, A61K 8/46, A61K 8/60, A61K 8/73, A61Q 5/02, A61K 8/11, B65D 65/46, C08K 5/053, C08L 5/04

(54) **ENSEMBLE COMPORTANT UN PRODUIT D HYGIÈNE DE DOUCHE OU DE BAIN ENVELOPPÉ DANS UN FILM HYDROSOLUBLE FONCTIONNEL**

(30) Priorité: 02.09.2021 FR 2109182
(71) Demandeur: POLYBRIDGE, 67000 Strasbourg (FR)
(72) Inventeur: XANTHOPOULOS, Timothée, 67000 STRASBOURG (FR); XANTHOPOULOS, Pascal, 67000 STRASBOURG (FR)
(74) Mandataire: Hege, Frédéric

(57) **Abrégé**

La présente invention concerne un ensemble comportant un produit d'hygiène de douche ou de bain (1) et un film (2), dans lequel ledit produit d'hygiène de douche ou de bain (1) est enveloppé dans ledit film (2), caractérisé en ce que ledit film (2) est soluble dans l'eau, et en ce que ledit film (2) comprend au moins
- un composant principal constitué d'alginate de sodium, dont la teneur en poids dans ledit film est supérieure ou égale à 25%, et
- un agent tensio-actif.

La présente invention concerne également une utilisation d'un ensemble selon l'invention.

## Description

La présente invention se situe dans le domaine des produits d'hygiène de douche ou de bain. Elle concerne plus particulièrement un produit d'hygiène de douche ou de bain enveloppé dans un film hydrosoluble apte à lui conférer des propriétés moussantes et émulsifiantes.

Les produits d'hygiène de douche ou de bain sont habituellement vendus dans des contenants en plastique. Ces contenants plastiques sont nocifs pour l'environnement, et sont lourds au transport et encombrants à domicile.

Le document WO2018 propose un film permettant d'emballer un shampoing. Ceci permet de réduire le volume de contenants plastiques, mais n'est pas d'usage pratique.

Un objet de la présente invention est de proposer un produit d'hygiène de douche dont l'emballage est réalisé à base de produits biodégradables et non nocifs pour l'environnement.

Un autre objet de la présente invention est de proposer un emballage léger pour produit d'hygiène de douche ou de bain.

Un autre objet de la présente invention est de proposer un emballage pour produit d'hygiène de douche ou de bain qui apporte des fonctions moussante et émulsifiante au produit d'hygiène de douche ou de bain.

Un autre objet de la présente invention est de proposer un emballage pour produit d'hygiène de douche ou de bain qui apporte une fonction hydratante au produit d'hygiène de douche ou de bain.

La présente invention a pour objet de répondre au moins en partie aux objets précités en proposant un ensemble comportant un film hydrosoluble comportant un agent tensio-actif apte à apporter des propriétés moussantes et émulsifiantes à un produit d'hygiène. A cet effet, elle propose un ensemble comportant un produit d'hygiène de douche ou de bain et un film, dans lequel ledit produit d'hygiène de douche ou de bain est enveloppé dans ledit film, caractérisé en ce que ledit film est soluble dans l'eau, et en ce que ledit film comprend au moins :
- un composant principal constitué d'alginate de sodium, dont la teneur en poids dans ledit film est supérieure ou égale à 25%, et
- un agent tensio-actif.

Grâce à ces dispositions, le produit d'hygiène de douche ou de bain est enveloppé dans un film hydrosoluble, léger, et qui peut être entièrement biosourcé et biodégradable, donc non nocif pour l'environnement. De plus lors de sa dissolution dans l'eau, l'agent tensio-actif du film peut se mélanger au produit d'hygiène de douche ou de bain et lui apporter des propriétés émulsifiantes et/ou moussantes.

Selon d'autres caractéristiques :
- la teneur en poids dudit agent tensio-actif dans ledit film peut être comprise entre 1 et 30%, ce qui permet au film de conférer un bon niveau de propriétés émulsifiantes et moussantes au produit d'hygiène et de douche,
- l'agent tensio-actif peut être d'origine naturelle, de type non inonique ou anionique, ce qui permet au film d'être biodégradable et moins nocif pour le corps humain,
- la viscosité dynamique de l'alginate de sodium du composant principal du film peut être comprise entre 200 et 500 mPa s, ce qui participe à ce que le film ait de bonnes propriétés mécaniques de résistance et flexibilité,
- ledit film peut comprendre en outre un glycérol, dont la teneur en poids dans ledit film peut être comprise entre 10 et 35%, lui permettant de jouer le rôle de produit plastifiant pour obtenir un film fin résistant et flexible,
- ledit film peut comprendre en outre un agent de réticulation tel que le chlorure de magnésium, dont la teneur en poids dans ledit film peut être comprise entre 3 et 5%, ce qui améliore encore la résistance mécanique du film, le chlorure de magnésium permettant de conserver une bonne solubilité dans l'eau du film,
- ledit film peut comprendre en outre un agent épaississant, dont la teneur en poids dans ledit film peut être comprise entre 1 et 30%, ce qui permet de conférer des propriétés hydratantes au produit d'hygiène de bain ou de douche lors de la dissolution du film,
- l'agent épaississant peut être un alginate de sodium dont la viscosité dynamique peut être comprise entre 3000 et 6000 mPa s, ce qui est un mode de réalisation simple et efficace de l'invention,

La présente invention concerne également une utilisation d'un ensemble selon l'invention, dans laquelle ledit film est dissout au contact de l'eau, libérant ledit agent tensio-actif dudit film, ledit agent tensio-actif apportant des propriétés émulsifiantes et/ou moussantes audit produit d'hygiène de douche ou de bain en se mélangeant avec celui-ci.

Grâce à ces dispositions, le produit d'hygiène de douche ou de bain est enveloppé dans un film hydrosoluble, léger, et qui peut être entièrement biosourcé et biodégradable, donc non nocif pour l'environnement. De plus lors de sa dissolution dans l'eau, l'agent tensio-actif du film peut se mélanger au produit d'hygiène de douche ou de bain et lui apporter des propriétés émulsifiantes et moussantes.

Selon d'autres caractéristiques :
- la dissolution du film peut libérer un agent épaississant contenu dans le film, celui-ci apportant des propriétés hydratantes audit produit d'hygiène de douche ou de bain en se mélangeant avec celui-ci, ce qui permet d'avoir un film dans le cadre de la présente invention apte à apporter des propriétés hydratantes à un produit d'hygiène de douche ou de bain.

La présente invention sera mieux comprise à la lecture de la description détaillée qui fait suite, en référence aux figures annexées dans lesquelles :
[Fig. 1] La fig. 1 est une vue schématique d'un ensemble selon un mode de réalisation préféré de l'invention.
[Fig. 2] La fig. 2 est une vue au microscope de micelles lors de l'exécution du deuxième test,
[Fig. 3] La fig. 3 est une vue en coupe de l'angle de contact entre une goutte d'eau et la peau d'un utilisateur.

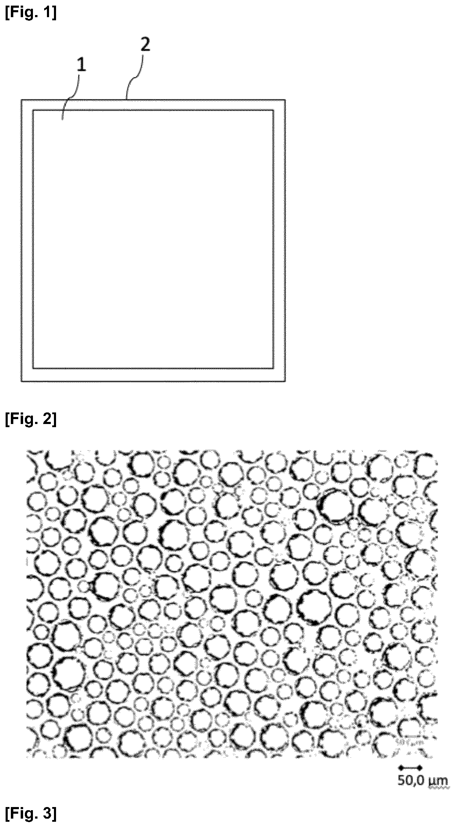

L'ensemble selon l'invention, illustré en fig. 1, comporte un produit d'hygiène de douche ou de bain 1 et un film 2, le produit d'hygiène de douche ou de bain 1 étant enveloppé dans le film 2.

Dans le cadre de la présente invention, le terme produit d'hygiène de douche ou de bain désigne tout produit d'hygiène corporelle liquide ou solide destiné à être utilisé sous la douche ou dans le bain. Il désigne par exemple un savon liquide ou solide, un gel douche, une huile de douche, un shampoing liquide ou solide, un après-shampoing, un avant-shampoing, ou encore un démêlant pour cheveux.

Le film 2 est soluble dans l'eau. La solubilité d'un film dans l'eau peut être mesurée de la façon suivante : un morceau de film (5 cm x 5 cm, découpé avec un emporte-pièce) est fixé sur un porte diapositive puis est plongé dans un bêcher de 1000 ml de capacité, contenant de 600 ml d'eau distillée à 20°C sous agitation, avec un barreau magnétique, à 300 tr/min. Le temps que met le film à se percer est mesuré. Le délitement du film entraîne la formation de particules de film. Le test dure 5 minutes au bout desquelles les particules sont passées dans un tamis de 0,5 mm pour vérifier la taille des particules. Lorsqu'il n'y a pas de particules dans le tamis, le film est considéré comme hydrosoluble.

Dans le cadre de la présente invention, on considère notamment que le film se dissout en quelques minutes, par exemple en moins de 3 minutes, lorsqu'il est au contact d'une eau à une température de bain ou de douche. Cette température peut être comprise entre 15 et 40°C, par exemple 37°C pour un bain chaud, ou par exemple 18°C pour une douche froide.

Le film 2 comprend un composant principal constitué d'alginate de sodium. La teneur en poids du composant principal dans le film 2 est supérieure ou égale à 25%, de préférence comprise entre 30 et 50%. L'alginate de sodium du composant principal a de préférence une viscosité dynamique comprise entre 200 et 500 mPa s, ce qui permet d'obtenir un film souple et résistant. Il peut par exemple s'agir du « Vivapharm » (marque déposée) Alginate PH R5 ou équivalent.

Dans le cadre de la présente demande, les valeurs de viscosité dynamique correspondent aux valeurs mesurées par la méthode Brookfield.

Le fim 2 comprend également un agent tensio-actif.

L'agent tensio-actif peut être de type non ionique. Il s'agit alors par exemple d'un polyoxyéthylène, un alcanolamide, un glucoside ou encore un oligopeptide, par exemple un alkylphénol éthoxylé, un alcool éthoxylé, un decyl glucoside, un alcool cetyl, un glutamate, un lauryl glucoside, ou un coco glucoside.

Le tensio-actif peut également être anionique tel qu'un sel d'acide carboxylique, de lipoaminoacides, de lipo-oligopeptides, un dérivé sulfoné ou un dérivé sulfaté, par exemple un lauryl sulfate de sodium (SLS), un lauryl sulfate d'ammonium (ALS) ou un de ses dérivés ethoxylés, un laureth sulfate de sodium (SLES), ou encore un tensio-actif d'acide sulfonique tels qu'un taurate, un iséthionate, un sulfonate d'oléfine, ou un sulfosuccinate.

Le tensio-actif peut encore être de type cationique, par exemple une amine, une alkylimidazoline, une amine alkoxylée, un behentrimonium methosulfate (BTMS), ou encore un composé d'ammonium quaternaire (aussi appelé Quats).

Le tensio-actif peut être de type amphotère, tel qu'une bétaïne, ou un dérivé d'acide aminé ou d'imidazole, par exemple la bétaïne cocamidopropyl.

La teneur en poids de l'agent tensio-actif dans le film 2 est de préférence comprise entre 1 et 30%, de préférence entre 5 et 25%.

La présence dans le film 2 de l'agent tensio-actif permet, lorsque le film 2 se dissout au contact de l'eau, de conférer au produit d'hygiène de douche ou de bain 1 des propriétés moussantes et émulsifiantes. La mousse facilite la répartition du produit d'hygiène de douche ou de bain 1 sur la peau et évite de multiplier les frottements sur le corps ou le cuir chevelu. Les propriétés émulsifiantes confèrent quant à elles une action lavante plus efficace au produit d'hygiène de douche ou de bain 1.

Un agent tensio-actif d'origine naturelle tel qu'un agent non ionique de la famille des glucosides, comme le coco-glucoside, ou un agent anionique tel que le sodium cocoyl glutamate, a l'avantage d'être plus doux et naturel que les tensio-actifs synthétiques utilisés habituellement dans les produits d'hygiène de douche ou de bain. De plus le composant principal est non nocif pour la peau et pour l'environnement.

L'ensemble selon l'invention peut être utilisé de la manière suivante : le film 2 se dissout au contact d'une eau à une température supérieure ou égale à 20°C, libérant l'agent tensio-actif. L'agent tensio-actif apporte alors des propriétés émulsifiantes et/ou moussantes au produit d'hygiène de douche ou de bain 1 en se mélangeant avec celui-ci.

Le film 2 peut encore comprendre un agent épaississant, dont la teneur en poids dans le fim 2 est par exemple comprise entre 1 et 30%. Cet épaississant est par exemple un alginate de sodium dont la viscosité dynamique est supérieure à 2500 mPa s, de préférence comprise entre 3000 et 6000 mPa s. Il peut par exemple s'agir du « Vivastar » (marque déposée) CS 052 Alginate ou équivalent.

La présence dans le film 2 de l'agent épaississant permet, lorsque le film 2 se dissout au contact de l'eau, de conférer au produit d'hygiène de douche ou de bain 1 des propriétés hydratantes en se mélangeant à celui-ci.

Le film 2 comprend de préférence un produit plastifiant, dont la teneur en poids dans le film 2 est par exemple comprise entre 10 et 35%. Le produit plastifiant permet de conférer au film les propriétés mécaniques voulues de résistance et de flexibilité. Le produit plastifiant peut être un glycérol, de préférence biosourcé, afin d'être moins nocif pour le corps humain et biodégradable.

Le film 2 comprend de préférence un agent de réticulation, dont la teneur en poids dans le film 2 est par exemple comprise entre 3 et 5%. L'agent de réticulation permet d'augmenter la résistance mécanique du film 2. L'agent de réticulation peut être par exemple un chlorure de calcium, ou de préférence un chlorure de magnésium qui présente l'avantage de pouvoir produire un film plus soluble dans l'eau.

### Exemples

Un shampoing liquide 1 est enveloppé dans plusieurs films différents à base d'alginate de sodium. Le film comparatif A, qui ne comporte pas d'agent tensio-actif, n'est pas un film selon l'invention. Les films B, C, D et E comportent chacun un agent tensio-actif, ils sont réalisés selon l'invention.

La composition des films est la suivante, avec les teneurs en poids des composants respectifs, le poids total de chaque film étant de 10 g :
- Film comparatif A :
   ∘ 65% « Vivapharm » (marque déposée) Alginate PH R5,
   ∘ 25% glycérol,
   ∘ 10% « Vivastar » (marque déposée) CS 052 Alginate.
- Film B :
   ∘ 65% « Vivapharm » (marque déposée) Alginate PH R5,
   ∘ 25% glycérol,
   ∘ 5% « Vivastar » (marque déposée) CS 052 Alginate,
   ∘ 5% coco-glucoside.
- Film C :
   ∘ 68% « Vivapharm » (marque déposée) Alginate PH R5,
   ∘ 25% glycérol,
   ∘ 5% « Vivastar » (marque déposée) CS 052 Alginate,
   ∘ 2% coco-glucoside.
- Film D :
   ∘ 65% « Vivapharm » (marque déposée) Alginate PH R5,
   ∘ 25% glycérol,
   ∘ 5% « Vivastar » (marque déposée) CS 052 Alginate,
   ∘ 5% bétaïne cocamidopropyl.
- Film E :
   ∘ 68% « Vivapharm » (marque déposée) Alginate PH R5,
   ∘ 25% glycérol,
   ∘ 5% « Vivastar » (marque déposée) CS 052 Alginate,
   ∘ 2% bétaïne cocamidopropyl.
- Film F :
   ∘ 65% « Vivapharm » (marque déposée) Alginate PH R5,
   ∘ 25% glycérol,
   ∘ 5% « Vivastar » (marque déposée) CS 052 Alginate,
   ∘ 5% cocoyl glutamate de sodium.
- Film G :
   ∘ 68% « Vivapharm » (marque déposée) Alginate PH R5,
   ∘ 25% glycérol,
   ∘ 5% « Vivastar » (marque déposée) CS 052 Alginate,
   ∘ 2% cocoyl glutamate de sodium.
- Film H :
   ∘ 65% « Vivapharm » (marque déposée) Alginate PH R5,
   ∘ 25% glycérol,
   ∘ 5% Vivastar (marque déposée) CS 052 Alginate,
   ∘ 5% behentrimonium methosulfate (BTMS).
- Film I :
   ∘ 68% « Vivapharm » (marque déposée) Alginate PH R5,
   ∘ 25% glycérol,
   ∘ 5% Vivastar (marque déposée) CS 052 Alginate,
   ∘ 2% behentrimonium methosulfate (BTMS).
- Film J :
   ∘ 55% « Vivapharm » (marque déposée) Alginate PH R5,
   ∘ 20% glycérol,
   ∘ 15% « Vivastar » (marque déposée) CS 052 Alginate,
   ∘ 10% coco-glucoside.
- Film K :
   ∘ 35% « Vivapharm » (marque déposée) Alginate PH R5,
   ∘ 15% glycérol,
   ∘ 25% « Vivastar » (marque déposée) CS 052 Alginate,
   ∘ 25% coco-glucoside.
- Film L :
   ∘ 60% « Vivapharm » (marque déposée) Alginate PH R5,
   ∘ 25% glycérol,
   ∘ 15% coco-glucoside.

Le Vivapharm (marque déposée) Alginate PH R5 est un alginate de sodium de viscosité 200-500 mPa s utilisé comme composant principal.

Le Vivastar (marque déposée) CS 052 Alginate est un alginate de sodium de viscosité 3000-6000 mPa s utilisé comme agent épaississant.

Le glycérol est utilisé comme plastifiant.

Le coco-glucoside, le bétaïne cocamidopropyl, le cocoyl glutamate de sodium et le behentrimonium methosulfate (BTMS) sont utilisés comme agents tensio-actifs.

Le film comparatif A et les films selon l'invention B à L sont élaborés par une méthode semblable au « casting » : pour chaque film, les ingrédients sont dispersés dans 30 ml d'eau, puis chaque solution est disposée dans un récipients en polyoléfine. Après deux à trois heures de séchage à l'air libre, des films d'épaisseur de 40 à 50 µm sont formés.

Ingrédients pour 50 cl de shampoing liquide 1 :
- 20 g de savon de Marseille,
- 40 cl d'eau,
- 1 cuillère à soupe de bicarbonate de soude,
- 10 gouttes d'huile essentielle d'arbre à thé.
- 1 cuillère à soupe de miel,
- 1 cuillère à soupe d'huile de coco,

Le shampoing liquide 1 est préparé selon le protocole suivant :
- râpage du savon de Marseille,
- introduction des copeaux de savon et de l'eau dans un récipient métallique chauffé à feu moyen en remuant,
- refroidissement et durcissement de la préparation,
- ajout du bicarbonate de soude et de l'huile essentielle d'arbre à thé,
- ajout d'eau pour rendre le mélange moins compact,
- ajout de l'huile de coco,
- mélange jusqu'à obtenir la consistance voulue.

Un premier test a été réalisé pour évaluer le pouvoir moussant de l'ensemble selon l'invention. Il s'agit d'un test comparatif basé sur le shampoing liquide 1 décrit ci-dessus, dans lequel on compare les résultats du shampoing liquide 1 seul, du shampoing liquide 1 enveloppé dans le film comparatif A, et du shampoing liquide 1 enveloppé dans les films selon l'invention B à L.

Le protocole du premier test est le suivant :
- introduction dans un premier bêcher de 250 ml d'eau à 35°C + 20 ml de shampoing liquide 1 sans emballage,
- introduction dans un bêcher A, respectivement B à L, de 250 ml d'eau à 35°C + 20 ml de shampoing liquide 1 emballé dans le film comparatif A, respectivement le fim selon l'invention B à L.
- introduction d'un agitateur magnétique dans chacun des treize béchers,
- démarrage simultané des agitateurs,
- mesure du remplissage de chaque bêcher après 5 minutes d'agitation.

Les mesures suivantes ont été effectuées :
- le premier bêcher contient 260 ml de solution,
- le bécher A contient 260 ml de solution,
- le bécher B contient 285 ml de solution,
- le bécher C contient 280 ml de solution,
- le bécher D contient 285 ml de solution,
- le bécher E contient 280 ml de solution,
- le bécher F contient 295 ml de solution,
- le bécher G contient 290 ml de solution,
- le bécher H contient 275 ml de solution,
- le bécher I contient 270 ml de solution,
- le bécher J contient 290 ml de solution,
- le bécher K contient 300 ml de solution,
- le bécher L contient 295 ml de solution.

Les mesures étant identiques pour le premier bécher et le bécher A, le shampoing liquide 1 emballé dans le film comparatif A ne contenant pas d'agent tensio-actif n'a pas produit plus de mousse que le shampoing liquide 1 utilisé sans film 1. Le fim comparatif A, lors de sa dissolution dans l'eau, n'apporte pas de pouvoir moussant complémentaire au shampoing liquide 1.

De plus, un volume plus important a été mesuré dans les béchers B à L que dans le premier bécher ; on peut en déduire que le shampoing liquide 1 emballé dans l'un des films selon l'invention B à L a produit plus de mousse que le shampoing liquide 1 utilisé sans film 1, ou que le shampoing liquide 1 enballé dans le film comparatif A ne contenant pas d'agent tensio-actif. Ceci montre que l'agent-tension actif confère un effet moussant à l'ensemble selon l'invention.

Un volume plus important ayant été mesuré dans le bécher K que dans le bécher L, dans le bécher L que dans le bécher J, dans le bécher J que dans le bécher B, et dans le bécher B que dans le bécher C, le premier test montre que pour un agent tensio-actif donné, dans ce cas le coco-glucoside, la quantité de mousse apportée par l'utilisation de l'ensemble selon l'invention est d'autant plus importante que la teneur en poids de l'agent tensio-actif est importante. Ceci se vérifie aussi pour les autres agents tensio-actifs testés : pour le bétaïnecocamidopropyl, le bécher D est plus rempli que le bécher E, pour le cocoyl glutamate de sodium, le bécher F est plus rempli que le bécher G, et pour le behentrimonium methosulfate (BTMS), le bécher H est plus rempli que le bécher I.

Enfin, ce premier test permet d'observer que certains agents tensio-actifs ont un effet moussant plus puissant que d'autres, à teneur égale dans les films. Les mesures montrent que le cocoyl glutamate de sodium, agent tensio-actif de type anionique, a un pouvoir moussant plus important que le coco-glucoside, agent tensio-actif de type non ionique, lui-même ayant un pouvoir moussant plus important que le bétaïne cocamidopropyl, agent-tensio actif de type amphotère, lui-même ayant un pouvoir moussant plus important que le behentrimonium methosulfate (BTMS), agent tensio-actif de type cationique.

Ainsi lors de la conception d'un film selon l'invention, pour un effet moussant voulu, la teneur en poids minimale en agent tensio-actif pourra être plus élevée pour un agent tensio-actif de type cationique, par exemple au moins 5%, que pour un agent tensio-actif de type anionique, par exemple au moins 1%.

Un deuxième test a été réalisé pour évaluer le pouvoir émulsifiant de l'ensemble selon l'invention. Il s'agit d'un test comparatif basé sur le shampoing liquide 1 décrit ci-dessus, dans lequel on compare les résultats du shampoing liquide 1 seul, du shampoing liquide 1 enveloppé dans le film comparatif A, et du shampoing liquide 1 enveloppé dans les films selon l'invention B à L.

Le deuxième test est basé sur le fait qu'un produit émulsifiant ajouté à des produits non miscibles, tel que l'eau et l'huile, provoque une macroémulsion, c'est-à-dire la formation de petites gouttelettes d'huile dans l'eau appelées micelles. Les bulles et gouttes sont visualisées au microscope numérique Keyence VHX 5000 (voir illustration en fig. 2). La taille des micelles se situe entre 50 et 150 micromètres, elles sont visibles à l'œil nu. Le nombre de micelles d'huile est d'autant plus élevé, et leurs tailles plus petites, que le produit a un pouvoir émulsifiant important.

Le protocole du deuxième test est le suivant :
- introduction dans un premier bêcher, ainsi que des béchers A à L, de 250 ml d'eau et 8 gouttes d'huile colorée. Après agitation, on observe que l'huile ne se disperse pas dans l'eau,
- ajout de 10 ml de shampoing liquide 1 dans le premier bêcher,
- ajout de 10 ml de shampoing liquide 1 enveloppé dans le film comparatif A, respectivement B à L, dans les béchers A, respectivement B à L,
- introduction d'un agitateur magnétique dans chacun des treize béchers,
- démarrage simultané des treize agitateurs,
- les macroémulsions produites dans chaque bécher sont observées après 10 minutes d'agitation à l'aide d'un microscope optique. Une photographie représentative de l'ensemble des micelles est prise pour chacune des émulsions prélevées du premier bécher et des béchers A à L, et les images obtenues sont analysées à l'aide d'un logiciel de traitement permettant d'obtenir la distribution de la taille des micelles.

Les mesures suivantes ont été effectuées :
- dans le premier bêcher, la majorité des goutelettes a un diamètre avoisinant les 130 µm,
- dans le bécher A, la majorité des goutelettes a un diamètre avoisinant les 130 µm,
- dans le bécher B, la majorité des goutelettes a un diamètre avoisinant les 110 µm,
- dans le bécher C, la majorité des goutelettes a un diamètre avoisinant les 115 µm,
- dans le bécher D, la majorité des goutelettes a un diamètre avoisinant les 110 µm,
- dans le bécher E, la majorité des goutelettes a un diamètre avoisinant les 115 µm,
- dans le bécher F, la majorité des goutelettes a un diamètre avoisinant les 105 µm,
- dans le bécher G, la majorité des goutelettes a un diamètre avoisinant les 110 µm,
- dans le bécher H, la majorité des goutelettes a un diamètre avoisinant les 115 µm,
- dans le bécher I, la majorité des goutelettes a un diamètre avoisinant les 126 µm,
- dans le bécher J, la majorité des goutelettes a un diamètre avoisinant les 100 µm,
- dans le bécher K, la majorité des goutelettes a un diamètre avoisinant les 90 µm,
- dans le bécher L, la majorité des goutelettes a un diamètre avoisinant les 95 µm.

Les mesures étant identiques pour le premier bécher et le bécher A, le shampoing liquide 1 emballé dans le film comparatif A ne contenant pas d'agent tensio-actif n'a pas un pouvoir émulsifiant plus élevé que le shampoing liquide 1 utilisé sans film 1. Le fim comparatif A, lors de sa dissolution dans l'eau, n'apporte pas de pouvoir émulsifiant complémentaire au shampoing liquide 1.

De plus, des tailles micelles plus petites ont été mesurées dans les béchers B à L que dans le premier bêcher ; on peut en déduire que le shampoing liquide 1 emballé dans l'un des films selon l'invention B à L a un pouvoir émulsifiant plus élevé que le shampoing liquide 1 utilisé sans film 1, ou que le shampoing liquide 1 enballé dans le film comparatif A ne contenant pas d'agent tensio-actif. Ceci montre que l'agent-tension actif confère un effet émulsifiant à l'ensemble selon l'invention.

Des tailles de micelles plus petites ayant été mesurées dans le bécher K que dans le bécher L, dans le bécher L que dans le bécher J, dans le bécher J que dans le bécher B, et dans le bécher B que dans le bécher C, le deuxième test montre que pour un agent tensio-actif donné, dans ce cas le coco-glucoside, le pouvoir émulsifiant apporté par l'ensemble selon l'invention est d'autant plus importante que la teneur en poids de l'agent tensio-actif est importante. Ceci se vérifie aussi pour les autres agents tensio-actifs testés : pour le bétaïnecocamidopropyl, des tailles de micelles plus petites ont été mesurées dans le bécher D que dans le bécher E, pour le cocoyl glutamate de sodium, des tailles de micelles plus petites ont été mesurées dans le bécher F que dans le bécher G, et pour le behentrimonium methosulfate (BTMS), des tailles de micelles plus petites ont été mesurées dans le bécher H que dans le le bécher I.

Enfin, ce deuxième test permet d'observer que certains agents tensio-actifs ont un effet émulsifiant plus puissant que d'autres, à teneur égale dans les films. Les mesures montrent que le cocoyl glutamate de sodium, agent tensio-actif de type anionique, a un pouvoir émulsifiant plus important que le coco-glucoside, agent tensio-actif de type non ionique, lui-même ayant un pouvoir équivalent à celui du bétaïne cocamidopropyl, agent-tensio actif de type amphotère, lui-même ayant un pouvoir moussant plus important que le behentrimonium methosulfate (BTMS), agent tensio-actif de type cationique.

Ainsi lors de la conception d'un film selon l'invention, pour un effet émulsifiant voulu, la teneur en poids minimale en agent tensio-actif pourra être plus élevée pour un agent tensio-actif de type cationique, par exemple au moins 5%, que pour un agent tensio-actif de type anionique, par exemple au moins 1%.

Un troisième test a été réalisé pour évaluer le pouvoir hydrant de l'ensemble selon l'invention lorsqu'il comporte un agent épaississant. Il s'agit d'un test comparatif basé sur le shampoing liquide 1 décrit ci-dessus, dans lequel on compare les résultats du shampoing liquide 1 seul, du shampoing liquide 1 enveloppé dans le film comparatif A, et du shampoing liquide 1 enveloppé dans les films selon l'invention B, D, F, H, J, K et L .

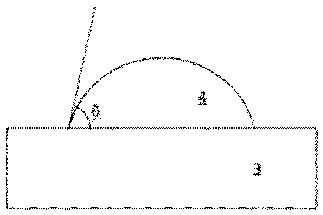

Lors du troisième test, L'efficacité hydratante du film hydrosoluble est évaluée par un test visuel de mouillabilié cutanée réalisé sur la surface plane de l'avant-bras d'un utilisateur. Le test visuel consiste à évaluer le degré d'étalement de l'eau sur la surface cutanée de l'avant-bras afin de révéler sa tendance hydrophobe ou hydrophile. Pour ce faire, un angle de contact θ peut être visualisé lorsqu'une goutte d'un liquide est déposée sur la surface cutanée. Comme illustré en fig. 3 , sur une vue en coupe transversale à la surface de la peau 3 et passant par le centre de la goutte 4, l'angle de contact θ est l'angle entre la surface de la peau et la droite tangeante à la goutte en son point de contact avec la peau. Une fois formée sur la surface, la goutte peut garder sa forme ou s'étaler, ce qui augmente la surface de contact. Une surface cutanée non traitée est à tendance hydrophobe : l'angle de contact θ est alors proche de 90° au niveau de l'avant-bras. Pour une goutte posée sur une peau hydraté, l'angle de contact θ diminue.

Le protocole du troisième test est le suivant, et identique pour le film comparatif A et chaque film B, D, F, H, J, K et L :
- introduction dans un bécher de 49,5 g d'eau à 30 °C,
- ajout dans le bécher d'un morceau de 5,5g du film, préalablement découpé en bandes,
- introduction d'un agitateur magnétique dans le bêcher,
- démarrage de l'agitateur, jusqu'à dissolution complète du film,
- imprégnation d'un gant de toilette de la solution contenue dans le bêcher,
- application statique du gant de toilette sur une zone à traiter de l'avant-bras d'un utlisateur pendant deux minutes,
- séchage de l'avant-bras à l'aide d'une serviette,
- dépôt d'une goutte d'eau sur la zone traitée de l'avant-bras,
- observation du degré d'étalement de la goutte d'eau, estimation de l'angle de contact θ.

Les deux dernières étapes du protocole sont également appliquées sur une zone non traitée de l'avant-bras de l'utilisateur.

Les mesures suivantes ont été effectuées :
- zone non traitée : l'angle de contact θ est proche de 90°,
- zone traitée avec le film comparatif A : l'angle de contact θ est d'environ 65°,
- zone traitée avec le film B : l'angle de contact θ est d'environ 70°,
- zone traitée avec le film D : l'angle de contact θ est d'environ 70°,
- zone traitée avec le film F : l'angle de contact θ est d'environ 70°,
- zone traitée avec le film H : l'angle de contact θ est d'environ 70°,
- zone traitée avec le film J : l'angle de contact θ est d'environ 60°,
- zone traitée avec le film K : l'angle de contact θ est d'environ 50°,
- zone traitée avec le film L : l'angle de contact θ est d'environ 80°,

L'angle de contact étant plus important avec le film L, qui ne contient pas d'agent épaississant, qu'avec le film comparatif A et les films B, D, F, H, J et K, qui contiennent un agent épaississant, les résultat du troisième test montrent que les zones traitées avec un film comportant un agent épaississant sont mieux hydratées que les zones traitées avec un film qui ne comporte pas d'agent épaississant. L'agent épaississant apporte donc bien une propriété hydratante aux films selon l'invention, et même au film comparatif A qui ne comporte pas d'agent tensio-actif.

De plus l'angle de contact θ étant plus faible pour la surface traitée avec le film K qu'avec le film J, avec le film J qu'avec le film comparatif A, et avec le film comparatif A qu'avec les films B, D, F et H, il est montré que les propriétés hydratantes sont d'autant plus importantes que la teneur du film en agent épaississant est élevée. Ceci se vérifie également avec le film comparatif A, et est donc indépendant de la présence de l'agent tensio-actif.

Bien que la description ci-dessus se base sur des modes de réalisation particuliers, elle n'est nullement limitative de la portée de l'invention, et des modifications peuvent être apportées, notamment par substitution d'équivalents techniques ou par combinaison différente de tout ou partie des caractéristiques développées ci-dessus.

## Revendications

1. Ensemble comportant un produit d'hygiène de douche ou de bain (1) et un film (2), dans lequel ledit produit d'hygiène de douche ou de bain (1) est enveloppé dans ledit film (2), **caractérisé en ce que** ledit film (2) est soluble dans l'eau, et **en ce que** ledit film (2) comprend au moins :
- un composant principal constitué d'alginate de sodium, dont la teneur en poids dans ledit film est supérieure ou égale à 25%, et
- un agent tensio-actif.

2. Ensemble selon la revendication précédente, dans lequel la teneur en poids dudit agent tensio-actif dans ledit film (2) est comprise entre 1 et 30%.

3. Ensemble selon l'une des revendications précédentes, dans lequel l'agent tensio-actif est d'origine naturelle, de type non ionique ou anionique.

4. Ensemble selon l'une des revendications précédentes, dans lequel la viscosité dynamique de l'alginate de sodium du composant principal du film (2) est comprise entre 200 et 500 mPa s.

5. Ensemble selon l'une des revendications précédentes, dans lequelle ledit film (2) comprend en outre un glycérol, dont la teneur en poids dans ledit film (2) est comprise entre 10 et 35%.

6. Ensemble selon l'une des revendications précédentes, dans lequelle ledit film (2) comprend en outre un agent de réticulation tel que le chlorure de magnésium, dont la teneur en poids dans ledit film (2) est comprise entre 3 et 5%.

7. Ensemble selon l'une des revendications précédentes, dans lequelle ledit film (2) comprend en outre un agent épaississant, dont la teneur en poids dans ledit film (2) est comprise entre 1 et 30%.

8. Ensemble selon la revendication précédente, dans lequel l'agent épaississant est un alginate de sodium dont la viscosité dynamique est comprise entre 3000 et 6000 mPa s.

9. Utilisation d'un ensemble selon l'une des revendications précédentes, dans laquelle ledit film (2) est dissout au contact de l'eau, libérant ledit agent tensio-actif dudit film, ledit agent tensio-actif apportant des propriétés émulsifiantes et/ou moussantes audit produit d'hygiène de douche ou de bain (1) en se mélangeant avec celui-ci.

10. Utilisation selon la revendication précédente d'un ensemble selon l'une des revendications 7 à 8, dans laquelle la dissolution du film (2) libère ledit agent épaississant, celui-ci apportant des propriétés hydratantes audit produit d'hygiène de douche ou de bain (1) en se mélangeant avec celui-ci.
